# EUROPEAN PATENT SPECIFICATION

(11) **EP 3 052 191 B1**
(45) Date of publication and mention of the grant of the patent: **18.03.2020**
(21) Application number: 14851149.6
(22) Date of filing: 23.09.2014
(51) Int. Cl.: C12N 5/0793, A61P 25/28, C12N 5/07, A61K 35/30, G01N 33/50, C12N 5/0735, C12N 5/079

(54) **DIRECTED DIFFERENTIATION OF ASTROCYTES FROM HUMAN PLURIPOTENT STEM CELLS FOR USE IN DRUG SCREENING AND THE TREATMENT OF AMYOTROPHIC LATERAL SCLEROSIS (ALS)**
GEZIELTE DIFFERENZIERUNG VON ASTROZYTEN AUS MENSCHLICHEN PLURIPOTENTEN STAMMZELLEN ZUR VERWENDUNG IM DROGENSCREENING UND ZUR BEHANDLUNG VON AMYOTROPHER LATERALSKLEROSE (ALS)
DIFFÉRENCIATION DIRIGÉE D'ASTROCYTES À PARTIR DE CELLULES SOUCHES PLURIPOTENTES HUMAINES POUR UTILISATION DANS LE CRIBLAGE DE MÉDICAMENTS ET LE TRAITEMENT DE LA SCLÉROSE LATÉRALE AMYOTROPHIQUE (SLA)

(30) Priority: 01.10.2013 US 201361885018 P; 17.06.2014 US 201462013003 P
(43) Date of publication of application: 10.08.2016
(73) Proprietor: Kadimastem Ltd., 74140 Nes-Ziona (IL)
(72) Inventor: IZRAEL, Michal, 76620 Rechovot (IL); REVEL, Michel, 76302 Rechovot (IL); HASSON, Arik, 55401 Kiryat-Ono (IL); MOLAKANDOV, Kfir, Yehud (IL); CHEBATH, Judith, 76286 Rechovot (IL)
(74) Representative: Straus, Alexander
(86) International application number: PCT/IL2014/050846
(87) International publication number: WO 2015/049677

(56) References cited:
- WO-A1-2007/025306
- WO-A1-2013/076726
- WO-A2-2010/042669
- WO-A2-2010/042669
- K GUPTA ET AL: "Human embryonic stem cell derived astrocytes mediate non-cell-autonomous neuroprotection through endogenous and drug-induced mechanisms", CELL DEATH AND DIFFERENTIATION., vol. 19, no. 5, 1 May 2012 (2012-05-01), pages 779-787, XP055340232, GB ISSN: 1350-9047, DOI: 10.1038/cdd.2011.154
- A. SERIO ET AL: "Astrocyte pathology and the absence of non-cell autonomy in an induced pluripotent stem cell model of TDP-43 proteinopathy", PROCEEDINGS OF THE NATIONAL ACADEMY OF SCIENCES, vol. 110, no. 12, 11 February 2013 (2013-02-11), pages 4697-4702, XP055340238, US ISSN: 0027-8424, DOI: 10.1073/pnas.1300398110
- WOEHRLING ET AL: "Development of a neurotoxicity test-system, using human post-mitotic, astrocytic and neuronal cell lines in co-culture", TOXICOLOGY IN VITRO, ELSEVIER SCIENCE, GB, vol. 21, no. 7, 1 October 2007 (2007-10-01), pages 1241-1246, XP022340063, ISSN: 0887-2333, DOI: 10.1016/J.TIV.2007.04.011
- Efthymiou A: "Functional screening assays with neurons generated from pluripotent stem cell-derived neural stem cells", Journal of Biomolecular Screening Society for Laboratory Automation and Screening, 9 September 2013 (2013-09-09), pages 32-43, XP055340253, Retrieved from the Internet: URL:http://journals.sagepub.com/doi/pdf/10 .1177/1087057113501869 [retrieved on 2017-01-30]
- K. MEYER ET AL: "Direct conversion of patient fibroblasts demonstrates non-cell autonomous toxicity of astrocytes to motor neurons in familial and sporadic ALS", PROCEEDINGS OF THE NATIONAL ACADEMY OF SCIENCES, vol. 111, no. 2, 30 December 2013 (2013-12-30), pages 829-832, XP055340269, US ISSN: 0027-8424, DOI: 10.1073/pnas.1314085111
- ROYBON, LAURENT ET AL.: 'Human stem cell -derived spinal cord astrocytes with defined mature or reactive phenotypes.' CELL REPORTS vol. 4, no. 5, 2013, pages 1035 - 1048., XP055331648 Retrieved from the Internet: <URL:http://www. cell .com/ cell - reports/pdf/S2211-1247(13)00313-6.pdf> [retrieved on 2015-01-13]

## Description

### FIELD AND BACKGROUND OF THE INVENTION

The invention, in some embodiments thereof, relates to a method of identifying agents that affect human astrocytes functionality using *ex-vivo* differentiated pluripotent stem cells (PSC). In addition, the use of human astrocytes for the treatment of Amyotrophic Lateral Sclerosis (ALS) is also provided.

ALS is a motor neurons (MN) disease that is characterized by the loss of upper and lower MNs (Moloney et al., Front Neurosci 2014, 8:252). It affects around 30,000 individuals in United States, about 150,000 patients globally and around 400 individuals in Israel. 5-10% of the ALS cases are familial ALS, whereas the majority of ALS cases are sporadic with unknown causes. Transgenic mice and rats carrying the ALS- associated mutant human SOD1 genes recapitulate many features of the human disease. Important to note that, to date, no effective treatment is available except one FDA-approved compound, Rilutek (Riluzole) that only extends lifespan by a maximum of three months.

ALS is diagnosed at a time that a significant cell loss has already occurred. The length of MN's axon is up to a meter. The MN's axon connects the cell body to its target, the muscle. The ability to appropriately rewire and ensure functional connections after MN replacement therapy remains a daunting task with questionable results and there is no evidence to date that this will be possible in humans. Therefore, an attempt to spare further cell loss would have significant impact on MNs survival and patients' quality of life and longevity following diagnosis.

However, cellular abnormalities in ALS are not limited to MNs. There are numerous observations of astrocyte malfunctioning in ALS patients, both sporadic ALS (sALS) and familial (fALS). Co-culture in vitro of astrocytes from ALS patients with normal motor neurons seem to accelerate the death of the motor neurons.

There is thus a widely recognized need for producing human astrocytes, propagated and expanded from human PSC cells that address these deficiencies.

WO 2010042669 discloses co-culture compositions and methods described for identifying agents that modulate a cellular phenotype, particularly of neurons or pancreatic beta cells, where the methods include co-culturing differentiated cells, wherein at least one of the cell-types are derived from human induced pluripotent stem cells from a subject having or predisposed to a neurodegenerative or metabolic disorder.

Gupta et al. describe in Cell Death and Differentiation, vol. 19, no. 5, 1 May 2012, pages 779-787) a human embryonic stem cell (HESC)-based system to assess the scope and mechanism of human astrocyte-mediated neuroprotection. Activation of the transcription factor Nrf2 in human astrocytes by CDDO(TFEA) treatment induced expression of the glutamate-cysteine ligase (GCL) catalytic subunit, leading to enhanced GCL activity and glutathione production, and strong neuroprotection against H(2)O(2).

Serio et al. disclose in Proceedings of the National Academy of Sciences, vol. 110, no. 12, 11 February 2013, pages 4697-4702) a unique platform to address the question of cell autonomy in transactive response DNA-binding protein (TDP-43) proteinopathies. The authors generated functional astroglia from human induced pluripotent stem cells carrying an ALS-causing TDP-43 mutation and show that mutant astrocytes exhibit increased levels of TDP-43, subcellular mislocalization of TDP-43, and decreased cell survival.

Woehrling et al. disclose in Toxicology in vitro, Elsevier Science, GB, vol. 21, no. 7, 1 October, 2007, pages 1241-1246) a neurotoxicity test-system, using human post-mitotic, astrocytic and neuronal cell lines in co-culture. Functional endpoints examined included assays for cellular energy (ATP) and glutathione (GSH) levels, generation of hydrogen peroxide (H(2)O(2)) and caspase-3 activation.

Meyer et al. describe in Proceedings of the National Academy of Sciences, vol. 111, no. 2, 30 December, 2013, pages 829-832) a reproducible method to convert adult human fibroblasts from living ALS patients to induced neuronal progenitor cells and subsequent differentiation into astrocytes (i-astrocytes). Non-cell autonomous toxicity to motor neurons is found following coculture of i-astrocytes from familial ALS patients with mutation in superoxide dismutase or hexanucleotide expansion in C9orf72 (ORF 72 on chromosome 9) the two most frequent causes of ALS.

### SUMMARY OF THE INVENTION

According to one aspect of the present invention, there is provided a method of screening an agent for preventing or treating Amyotrophic Lateral Sclerosis (ALS) the method comprising::
(a) contacting a population of astrocytes, the astrocytes having been *ex-vivo* differentiated from pluripotent stem cells (PSC), with the agent;
(b) co-culturing the population of astrocytes of step (a) or a conditioned medium thereof with a population of neurons under stress selected from hypoxicity, oxidative stress, glutamate toxicity or AMPA/kainate toxicity; and
(c) quantifying an effect of said agent to enhance survival or neural function of the population of neurons.

According to some embodiments of the invention, the ratio of the population of astrocytes to neurons is greater than 1:1, 10:1, 100:1, 1000:1, or 10,000:1.

According to some embodiments of the invention, the astrocytes express each of GFAP, GLAST, AQP4, or a combination thereof.

According to some embodiments of the invention, the astrocytes display secretion of neurotrophic factors selected from the group consisting of BDNF, GDNF and VEGF.

According to some embodiments of the invention, the oxidative stress is selected from the group consisting of reactive oxygen species (ROS), hydrogen peroxide (H₂O₂), and any derivative thereof.

According to some embodiments of the invention, the quantifying is conducted by counting the number of neurons which are under apoptosis.

According to some embodiments of the invention, the apoptosis of said neurons is detected by Caspase-3a labeling, Annexin V, Tubulin-B3, HB9 or DAPI.

According to some embodiments of the invention, the agent is a small molecule.

Normal human progenitor astrocytes or astrocytes should provide an environment to prevent motor neuron death.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

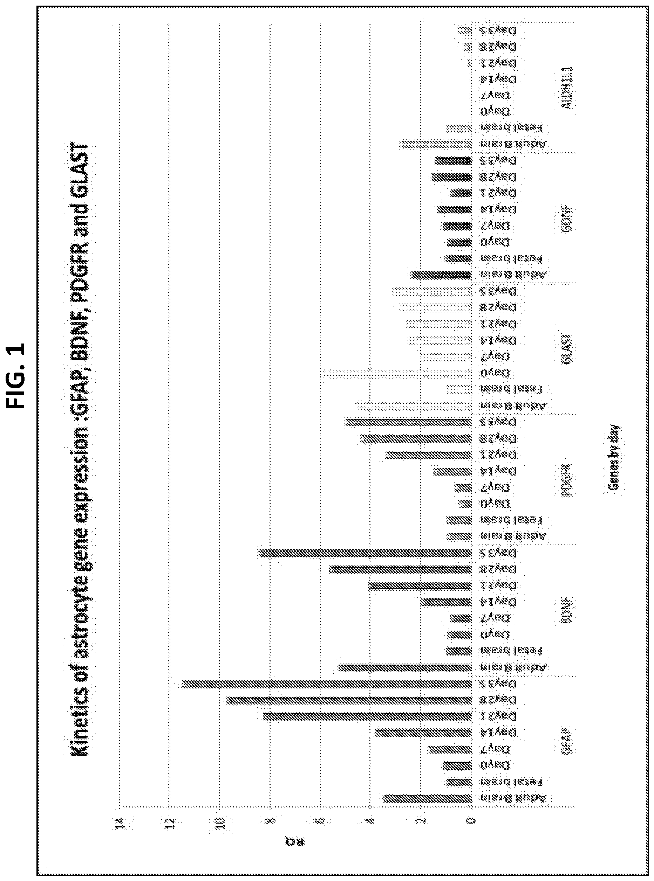

FIG. 1 illustrates the kinetics of astrocyte lineage gene expression by hESC derived astrocytes. Expression of day 0, 7, 14, 21, 28 and 35 for each gene is presented. Human adult and fetal brain were used as controls. The following colors orange, blue, green, yellow, red and light blue represent the gene expression kinetics of GFAP, BDNF, PDGFRα, GLAST, GDNF and ALDH1L1 respectively. For each gene fetal brain expression served as a reference gene (Relative Quantification, RQ=1).

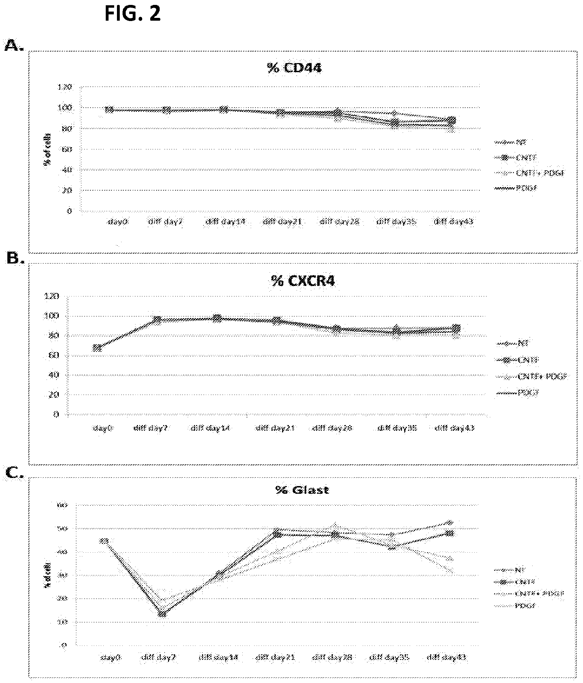

FIGs. 2A-C illustrate that hESC derived astrocytes express astrocytes markers. A. CD44, B.CXCR4 and C. GLAST were measured by FACS for 0, 7, 14, 21, 28, 35 and 43 days. PDGF and CNTF are factors known to promote APC commitment and differentiation. The effect of non-treated (NT), PDGF, CNTF and PDGF+CNTF treatments on hESC-APC differentiation was tested (% of marker expression). Blue line: NT, Red line: CNTF, Green line: CNTF+PDGF and Light blue: PDGF. The population which is obtained by hPSC derived astrocyte protocol results in an enriched population of astrocytes.

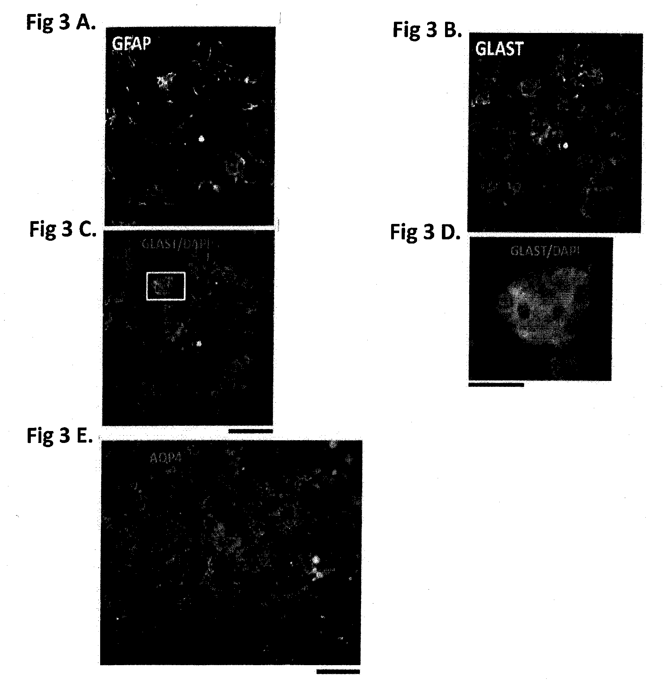

FIGs. 3A-E illustrate that hESC derived astrocytes express markers of mature astrocytes. A. Grey levels of GFAP positive cells. B. Grey levels of GLAST positive cells. C. Overlay montage image of A+B, in red GFAP positive cells, green GLAST positive cells and in Blue, Dapi positive cells. Yellow staining suggests co-staining for GFAP and GLAST. D. Enlargement of C insert (same remark regarding yellow staining). E. In green Aquaporin-4 positive cells (AQP4) and in red GLAST positive cells. A-C and E Scale bar: 100µm and D scale bar: 30µm.

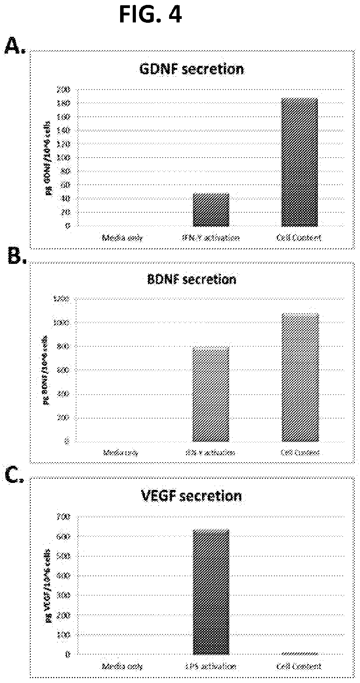

FIGs. 4A-C illustrate that hPSC derived astrocytes produce and secrete neurotrophic factors, as measured in Cell content and in media after astrocytes activation respectively. A. GDNF cell content and secretion after IFN-γ activation (in red). B. BDNF cell content and secretion after IFN-γ activation (in orange). C. VEGF cell content and VEGF secretion after LPS activation (in purple).

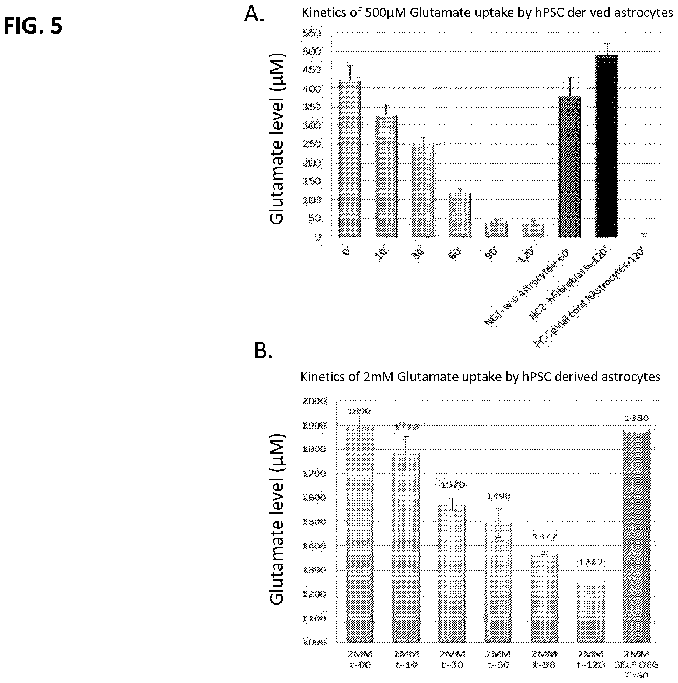

FIGs. 5A-B illustrate kinetics of 500µM and 2mM glutamate uptake by hESC derived astrocytes. A. From left to right (Green bars): 0', 10', 30', 60' 90' and 120' after addition of 500µM glutamate to hESC derived astrocytes at day 100 of differentiation. Light red bar: 1^{st} negative control, media with 500µM glutamate 60' without the presence of astrocytes. Dark red bar- 2^{nd} negative control, Levels of Glutamate in media grown with human fibroblasts after 120'. Right bar (blue): positive control Levels of Glutamate in media grown with human spinal cord derived astrocytes. B. From left to right (light green bars): 0', 10', 30', 60' 90' and 120' after addition of 2mM glutamate to hESC derived astrocytes at day 100 of differentiation. Blue bar: negative control, media with 2mM glutamate after 60' without the presence of astrocytes.

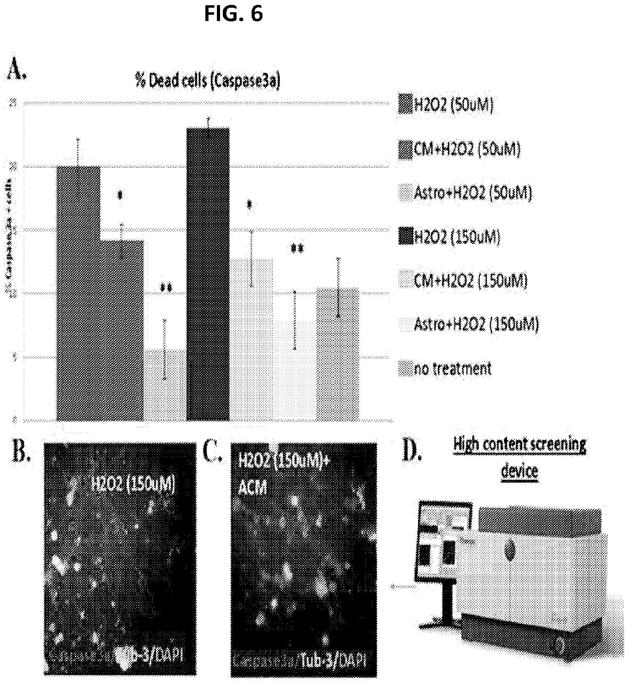

FIGs. 6A-D illustrate neuroprotective effect of human PSC derived astrocytes on Motor Neurons under oxidative stress.
   A. MNs were supplemented with H₂O₂ (50µM and 150µM) or left non-treated for 6 hours. During this period MN were left alone or supplemented with astrocytes supernatant (24 hour conditioned medium) or with astrocytes. A: % Caspase -3a in MN without H₂O₂ is represented by the grey bar (right). %Caspase-3a in MN incubated with H₂O₂ alone is represented by the red (50µM H₂O₂) and olive green bar (150µM H₂O₂), or after addition of astrocyte conditioned medium by the orange and pale green bars, and in the presence of astrocytes by the pink and pale green bar. In B and C Caspase -3 activation (red). Motor neurons are stained with beta3 tubulin (green) and nuclei with DAPI. D. An image of the high content analysis device used for image quantification and analysis.

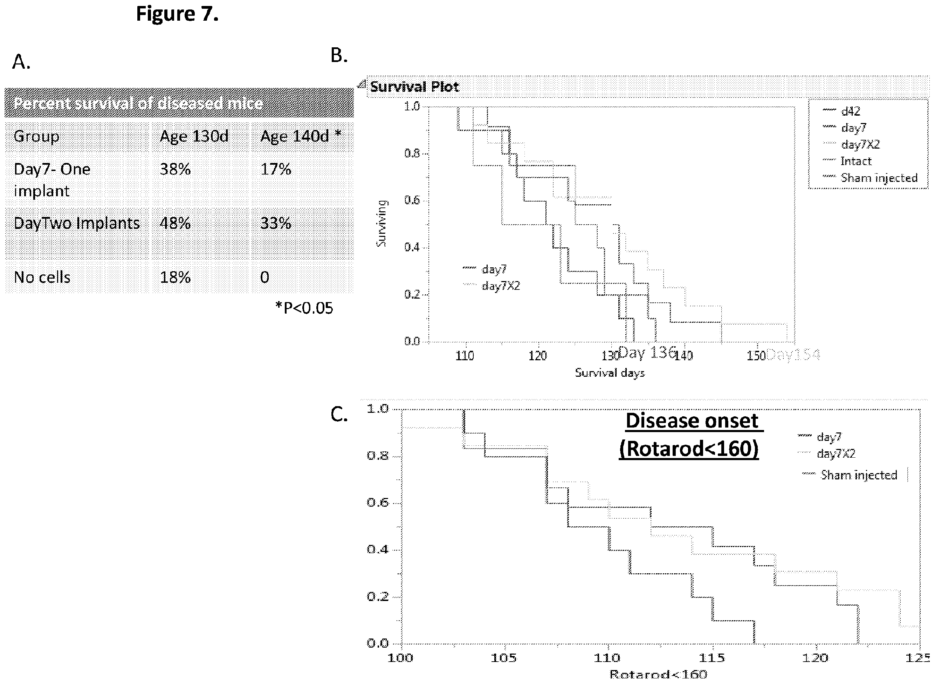

FIGs. 7A-C demonstrate that injection of human progenitor astrocytes (hAPC) increased survival of hSOD1 mice. A. Table comparing survival of hSOD1 mice in two time points (day 130 and day140). Survival of mice that were transplanted with "day7" cells (grown in the absence of growth factors for 7 days) was increased as shown in day140. B. Kaplan-Meir survival graph comparing all experimental groups. C. Kaplan-Meier graph generated for disease onset as measured by Rotarod<106 comparing all experimental groups (not accoding to invention).

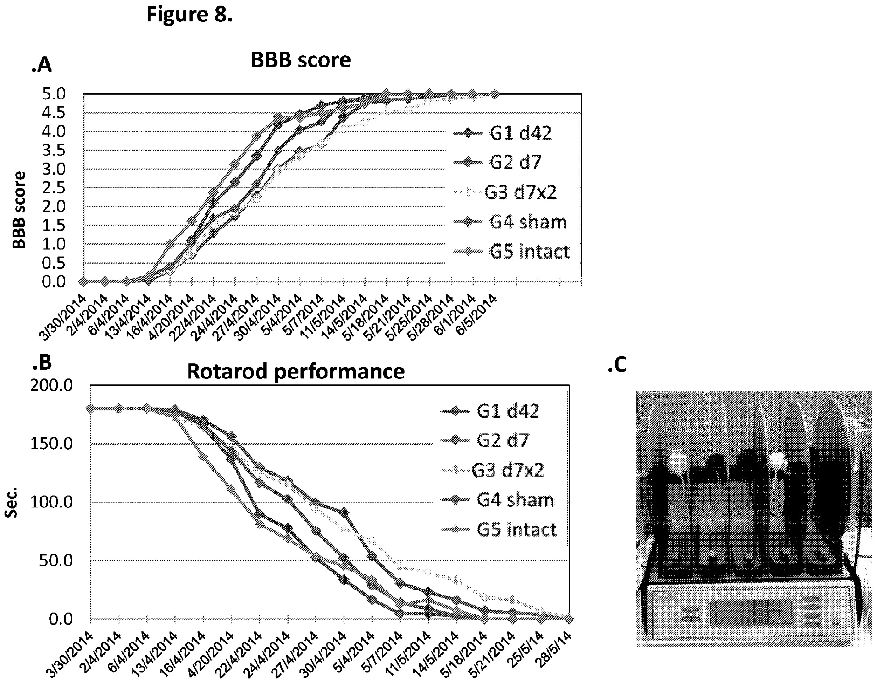

FIGs. 8A-C demonstrate that young human derived astrocytes increased motor performance in hSOD1 mice. A. Kinetics of disease progression as described by BBB score comparing all groups. 0=normal mice 5= paralyzed mice. B. Kinetics of disease progression as described by rotarod score comparing all experimental groups, 180 sec= normal mice 0=mouse that can't hold the rod. C. mice on Rotarod apparatus.

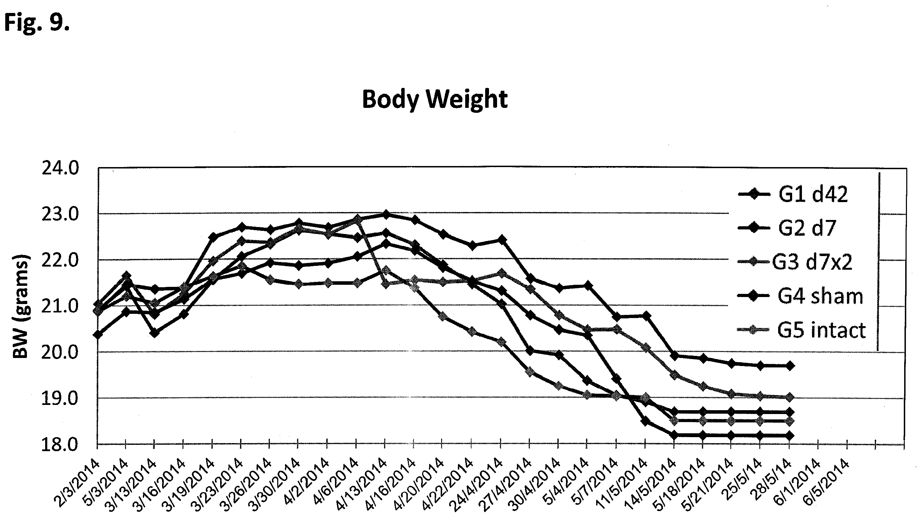

FIG. 9 demonstrates that young human derived astrocytes maintained transplanted hSOD1 body weight. Kinetics of changes in body weight throughout disease progression comparing all groups.

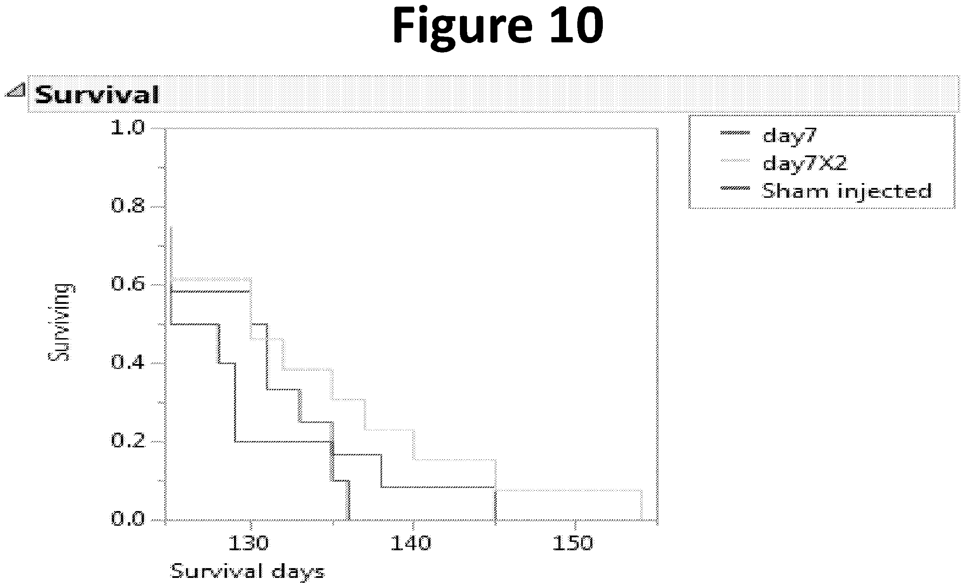

FIG. 10 illustrates Kapaln Meier Log rank statistical analysis. Log Rank survival analysis was conducted on 50% of the population.

### DESCRIPTION OF SPECIFIC EMBODIMENTS OF THE INVENTION

The present invention, in some embodiments thereof, relates to a method of identifying agents that affect human astrocytes functionality using *ex-vivo* differentiated pluripotent stem cells (PSC).

To obtain large amounts of human astrocytes the inventors of the present invention propose to differentiate them from human pluripotent stem cells (PSC) cells that can be propagated indefinitely before differentiation.

The present invention further disclosed a unique and robust protocol for generating a highly homogenous population of astrocytes (>90% GFAP, S100b) from human pluripotent stem cells (hPSC). The differentiation protocol together with the scalable culturing technology, allow the production of large quantities of astrocytes in vitro. These hPSC-derived astrocytes exhibit similar gene expression pattern as primary human astrocytes as well as functional properties including: I) Secretion of neurotrophic factors that protect motor neurons (BDNF, GDNF and VEGF). II) Capacity of glutamate uptake and III) Protection of neurons from oxidative stress in vitro. These hPSC derived astrocytes can be kept frozen and used upon need.

The therapeutic properties of said human astrocytes were assessed in-vivo, for that aim the cells were transplanted intrathecally into G93A high copy number hSOD1 mice (a mouse model for ALS disease). Human astrocytes transplantation resulted in significant improvement (P<0.05) in motor performance in all functional tests. In addition, positive effect on survival and disease duration was observed in transplanted mice.

ALS is characterized by the death of motor neurons in the cortex, the brain stem, and the spinal cord. ALS is familial in about 5-10% of the cases and is sporadic in the rest. The course of the disease is indistinguishable between familial and sporadic ALS. The disease manifests itself late in life at an average age of 56 years, and once diagnosed, leads to complete paralysis and death within 2-5 years. In a subset of the familial patients, mutations have been found in a gene coding for copper-zinc superoxide dismutase 1 (SOD1). SOD1 is a cytosolic enzyme involved in detoxification of free radicals. Further, overproduction of pathogenic human SOD1 protein encoding alleles in motor neurons leads to late-onset, progressive neurodegenerative disease. Studies have led to the identification of intrinsic pathogenic characteristics of ALS motor neurons, including the formation of protein aggregates, cytoskeletal abnormalities, proteosome dysfunction and increased sensitivity to cell death signals. Additionally, overproduction of pathogenic human SOD1 protein encoding alleles in other cells often found associated with motor neurons in vivo, such as but not limited to glial cells (e.g. astrocytes, Schwann cells, etc.) may result in non-cell autonomous motor neuron pathologies. Examples of a pathogenic SOD1 protein encoding allele include but are not limited to SOD1G93A, SOD1G85R, and SOD1G37R.

Neurodegenerative diseases such as ALS, Alzheimer's disease and Parkinson's disease, are untreatable conditions that collectively represent a major healthcare burden. Improved understanding of the biology of these diseases is required in order to develop neuroprotective and ultimately reparative treatments. Until recently, neurodegenerative diseases have been largely regarded as exclusively neuronal disorders. However, recent findings have challenged this concept, implicating non-cell-autonomous mechanisms of neurodegeneration mediated by astrocytes in acute and chronic disorders. Human stem cell biology allows the modeling of human astrocyte-neuronal interaction under physiological and injury paradigms. Although there are robust platforms for the generation of neural stem cells and functional neurons, the generation of enriched and functional human astrocytes is comparatively understudied. The mechanisms by which human astrocytes might protect diseased neurons and increase their survival are hypothesized to include: prevention of glutamate neurotoxicity, secretion of neurotrophic factors (NTF) such as BDNF and GDNF, and neurovascular modification by VEGF secretion.

The present invention is based in part on the generation of enriched and functional population of human pluripotent stem cells derived progenitor astrocytes and astrocytes in-vitro, and the establishment of a drug screening platform for ALS, in which the neuroprotective effect of human astrocytes on motor neurons under oxidative stress was tested.

In some embodiments of the present invention, methods and compositions are provided for the identification, characterization and optimization of lead compounds that exhibit neuroprotective effects and enhance human astrocytes functionality.

Agents which may be tested using the assay of the invention include small molecule agents, chemicals, peptides, proteins, polynucleotide agents (e.g. siRNA agents).

Contacting the cells with the agent can be performed by any *in-vitro* method known in the art including for example, adding the agent to the cells such that the agent is in direct contact with the cells. According to the invention, the cells are incubated with the agent. The conditions used for incubating the cells are selected for a time period/concentration of cells/concentration of agent/ratio between cells and agent and the like, which enable the agent to induce cellular changes, such as changes in transcription and/or translation rate of specific genes, which are analyzed. The cells may be contacted with the agent for at least 1 day, 3 days, 5 days, 7 days, 10 days, 14 days or at least 21 days

Candidate neuroprotective lead compounds may be added to the culture media of cells of the present invention and assayed for desirable properties. Compounds exhibiting desirable properties such as compounds that increase the number of viable motor neurons in a culture relative to the number of viable motor neurons in the absence of the candidate neuroprotective lead compound may be selected for further characterization and optimization. Compounds may be selected for their ability to increase the viability of motor neurons in a co-culture with human astrocytes, or in conditioned media thereof.

The high-throughput screens may also be used to identify compounds capable of correcting any disease-associated phenotype, e.g., survival, apoptosis, necrosis, axonal degeneration, axonal guidance, axonal morphology, dendritic morphology, receptor density, synaptogenesis, neurogenesis, synapse density, synaptic transmission, synaptic signaling, receptor trafficking, protein trafficking, protein aggregation, proteasome activity, receptor expression, oxidative stress (ROS), FGFR-signaling and FGF signaling.

Characterization and optimization of candidate lead compounds selected for their ability to modulate a cellular phenotype (e.g., neuroprotective effect) include but is not limited to further screening, generation of derivatives and analogues, analysis of Absorption Distribution, Metabolism, and Excretion (ADME) characteristics, safety trials, and efficacy trials. In particular, optimization efforts may involve developing compounds which are designed to cross the blood brain barrier.

Drug candidate compounds (or "test agents") may be individual small molecules of choice (e.g., a lead compound from a previous drug screen) or in some cases, the drug candidate compounds to be screened come from a combinatorial library, i.e., a collection of diverse chemical compounds generated by either chemical synthesis or biological synthesis by combining a number of chemical "building blocks." For example, a linear combinatorial chemical library such as a polypeptide library is formed by combining a set of chemical building blocks called amino acids in every possible way for a given compound length (i.e., the number of amino acids in a polypeptide compound). Millions of chemical compounds can be synthesized through such combinatorial mixing of chemical building blocks. Indeed, theoretically, the systematic, combinatorial mixing of 100 interchangeable chemical building blocks results in the synthesis of 100 million tetrameric compounds or 10 billion pentameric compounds.

As used herein, the term "survival" is meant any process by which a cell avoids death. The term survival, as used herein, also refers to the prevention of cell loss as evidenced by necrosis, apoptosis, or the prevention of other mechanisms of cell loss. Increasing survival as used herein indicates a decrease in the rate of cell death by at least 10%, 25%, 50%, 75%, 100%, or more relative to an untreated control. The rate of survival may be measured by counting cells capable of being stained with a dye specific for dead cells (e.g., propidium iodide) in culture.

The cells used in the assay of the present invention comprise astrocytes cells which have been *ex-vivo* differentiated from pluripotent stem cells.

For reference only, human embryonic stem cells can be isolated from human 5 blastocysts or delayed blastocyst stage (as described in WO2006/040763). Human blastocysts are typically obtained from human *in-vivo* preimplantation embryos or from *in-vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by immunosurgery, in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 1-2 weeks. For further details on methods of preparation human ES cells see Thomson et al., [U.S. Pat. No. 5,843,780; Science 282: 1145, 1998; Curr. Top. Dev. Biol. 38: 133, 1998; Proc. Natl. Acad. Sci. USA 92: 7844, 1995]; Bongso et al., [Hum Reprod 4: 706, 1989]; Gardner et al., [Fertil. Steril. 69: 84, 1998].

It will be appreciated that commercially available stem cells can also be used with this aspect of the disclosure. Human ES cells can be purchased from the NIH human embryonic stem cells registry (<http://escr.nih.gov>). Non-limiting examples of commercially available embryonic stem cell lines are BG01, BG02, BG03, SA01, TE03 (I3), TE04, TE06 (I6), HES-1, HES-2, HES-3, UC01, UC06, WA01, WA07 and WA09.

Stem cells used by the present invention can be also derived from induced pluripotent stem cells (IPSc).

Regardless of their origin, stem cells used in accordance with the present invention are at least 50 % purified, 75 % purified or at least 90 % purified. When human embryonic stem cell lines are used, the human ES cell colonies are separated from their feeder layer (x-ray irradiated fibroblast-like cells) such as by mechanical and/or enzymatic means to provide substantially pure stem cell populations.

Once human stem cells are obtained, they may be treated to differentiate into astrocytes. An exemplary method is described in the Materials and Methods section below. It will be appreciated however, that the present method contemplates additional methods for generating astrocytes, which are known in the art.

The culture medium used is selected according to the stem cell used. Thus, for example, a medium suitable for ES cell growth, can be, for example, DMEM/F12 (Sigma- Aldrich, St. Lewis, MO) or alpha MEM medium (Life Technologies Inc., Rockville, MD, USA), supplemented with supporting enzymes and hormones. These hormones can be for example insulin (ActRapid; Novo Nordisk, Bagsværd, DENMARK), progesterone and/or Apo transferrin (Biological Industries, Beit Haemek, Israel). Other ingredients are listed in the Examples section.

As used herein the phrase "retinoic acid" refers to an active form (synthetic or natural) of vitamin A, capable of inducing neural cell differentiation. Examples of retinoic acid forms which can be used in accordance with the invention include, but are not limited to, retinoic acid, retinol, retinal, 11-cis-retinal, all-trans retinoic acid, 13-cis retinoic acid and 9-cis-retinoic acid (all available at Sigma- Aldrich, St. Lewis, MO).

Retinoic acid may be used at a concentration range of 1-50 µM.

The culture medium may be further supplemented with growth factors, which may be present at least in part of the culturing period to promote cell proliferation and facilitate differentiation into the neuronal glial lineages Such growth factors may include for example, EGF (5-50 ng/ml) and bFGF (5-50 ng/ml) (R&D Systems, Minneapolis, MN, Biotest, Dreieich, Germany).

As used herein the phrase "neurospheres" refers to quasi-spherical clusters or spheres containing mainly neural stem cells and early multipotent progenitors that can differentiate into neurons, oligodendrocytes and astrocytes as well as other glial cells.

The cells are cultured until ripened neurospheres are formed.

As used herein the phrase "ripened neurospheres" refers to neurospheres in which some of the neural stem cells have differentiated to become specialized oligodendrocyte progenitors having acquired makers of the oligodendrocyte lineage (e.g. Sox10, Nkx2.2., NG2, A2B5), while others have differentiated to become neural progenitors or astrocytes progenitors.

The cells may be allowed to culture, for example for 10-30 (e.g., 20-30) days, at the end of which detached neurospheres are formed. The spheres, or cells dissociated therefrom, are then adhered to adherent substrates (e.g. matrigel or an extracellular matrix component (e.g., collagen, laminin and fibronectin) and subjected to further expansion with growth factors and eventually to differentiation after removal of growth factors on a cationic adherent substrate (e.g. poly-D- lysine or Polyornithine with fibronectin (FN) or adherent substrates (e.g. matrigel or an extracellular matrix component (e.g., collagen, laminin and fibronectin).

As used herein the terms "human astrocyte progenitor cells (hAPC)" or "human progenitor astrocytes" mean cells that can generate progeny that are mature astrocytes. Typically, the cells express some of the phenotypic markers that are characteristic of the astrocyte lineage.

The terms "administering" and "administration" refer to the process by which a therapeutically effective amount of astrocytes or medicament contemplated herein is delivered to a subject for prevention and/or treatment purposes. The astrocytes and medicaments are administered in accordance with good medical practices taking into account the subject's clinical condition, the site and method of administration, dosage, patient age, sex, body weight, and other factors known to physicians.

The term "treating" refers to reversing, alleviating, or inhibiting the progress of a disease, or one or more symptoms of such disease, to which such term applies. Treating includes the management and care of a subject at diagnosis or later. A treatment may be either performed in an acute or chronic way. Depending on the condition of the subject, the term may refer to preventing a disease, and includes preventing the onset of a disease, or preventing the symptoms associated with a disease. The term also refers to reducing the severity of a disease or symptoms associated with such disease prior to affliction with the disease. Such prevention or reduction of the severity of a disease prior to affliction refers to administration of astrocytes or medicament comprising same, to a subject that is not at the time of administration afflicted with the disease. "Preventing" also refers to preventing the recurrence of a disease or of one or more symptoms associated with such disease. An objective of treatment is to combat the disease and includes administration of the astrocytes to prevent or delay the onset of the symptoms or complications, or alleviating the symptoms or complications, or eliminating or partially eliminating the disease. The terms "treatment" and "therapeutically," refer to the act of treating, as "treating" is defined above.

The terms "subject", "individual", or "patient" are used interchangeably herein and refer to an animal including a warm-blooded animal such as a mammal.

As utilized herein, the term "healthy subject" means a subject, in particular a mammal, having no diagnosed or symptoms of ALS.

As used herein a "pharmaceutical composition" refers to a preparation of one or more of the active ingredients or cells described herein with other chemical components such as physiologically suitable carriers and excipients. The purpose of a pharmaceutical composition is to facilitate administration of cells or compound to an organism.

Herein the term "active ingredient" refers to an agent which enhance survival or neural function of the population of motor neurons.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

"Therapeutically effective amount" relates to the amount or dose of astrocytes or medicament thereof, that will lead to one or more desired effects, in particular, one or more beneficial effects. A therapeutically effective amount of a substance can vary according to factors such as the disease state, age, sex, and weight of the subject, and the ability of the substance to elicit a desired response in the subject. A dosage regimen may be adjusted to provide the optimum therapeutic response (e.g. beneficial effects, more particularly sustained beneficial effects). For example, several divided doses may be administered daily or the dose may be proportionally reduced as indicated by the exigencies of the therapeutic situation.

Induced multipotent and pluripotent stem cell lines are referred to as "induced stem cell lines" (iSC lines) herein. Induced pluripotent stem cells are referred to as iPS cells or iPSCs.

"Correcting" a phenotype, as used herein, refers to altering a phenotype such that it more closely approximates a normal phenotype.

Techniques for formulation and administration of drugs may be found in "Remington's Pharmaceutical Sciences," Mack Publishing Co., Easton, PA, latest edition, which is incorporated herein by reference.

As used herein the term "about" refers to ± 10 %.

The terms "comprises", "comprising", "includes", "including", "having" and their conjugates mean "including but not limited to".

The term "consisting of means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Various embodiments and aspects of the present invention as claimed in the claims section below find experimental support in the following examples.

### EXAMPLES

Reference is now made to the following examples, which together with the above descriptions illustrate some embodiments of the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### MATERIALS AND METHODS

### Generation of the astrocytes used for the assay

Human ES cell lines were cultured at 37 °C in 5 % CO₂ on feeder layers of human new-born foreskin fibroblasts (HEF). The growth medium (ES1) consisted of DMEM/F12 (Sigma), 14 % knockout serum replacement (KSR), nonessential amino acids (1/100), 0.1 mM beta-mercaptoethanol (all three from Invitrogen/Gibco), 1 mM Na pyruvate, 2 µg/ml heparin (Sigma) and 8 ng/ml human recombinant basic FGF (FGF-2; Preprotech). Five days after seeding, hES cell colonies were detached with 1.2 mg/ml collagenase IV (Worthington) for 45-90 minutes at 37 °C. The aggregates were further cultured as above, or subjected to differentiation as described (Izrael et al Mol Cell Neurosci 34, 310-323 (2007).
***Step 1-transition:*** For this transition step, hES cell colonies were transferred to tissue culture plates in medium T (Transition) consisting of 50 % (v/v) ES1 medium and 50 % (v/v) of ITTSPP/B27medium. ITTSPP/B27 is itself a mixture of one volume of DMEM/F12 containing 2 % B27 supplement (Invitrogen) and one volume of DMEM/F12 containing 25 µg/ml human insulin (ActRapid, Novo Nordisk), 50 µg/ml human Apo-transferrin (Biological Industries, Israel), 6.3 ng/ml progesterone, 10 µg/ml putrescine, 50 ng/ml sodium selenite and 40 ng/ml triiodothyronine (T3) (all from Sigma), with 100 U/ml penicillin and 100 µg/ml streptomycin. Medium T was supplemented by 20 ng/ml r-human EGF (R&D Systems) and 4 ng/ml r-human bFGF. After 1 day, the bulk of non-adherent hES cells colonies and aggregates were transferred to 6 cm bacterial (non-adherent) plates and grown in T medium supplemented with 20 ng/ml r-human EGF (R&D Systems) and 2 ng/ml r-human bFGF.
***Step 2: All-trans retinoic acid (ATRA) treatment:*** The medium was switched to ITTSPP/B27, with 20 ng/ml EGF and 10 µM ATRA additions. Medium was changed daily for 7 days.
***Step 3: Suspension culture:*** During this step, which allows for ripening of neurospheres (NS), the culture was continued in ITTSPP/B27 medium with 20 ng/ml EGF for 18 days. Medium was changed every other day.

### Neural stem cells proliferation and differentiation

***Step 4: Matrigel adhesion 1:*** Spheres/clusters were transferred to 6-well tissue culture plates that had been coated for 1.5 h at room temperature (RT) with BD Matrigel (growth factor reduced Matrigel, BD Biosciences) diluted 1:30 in DMEM/F12. The culture medium was ITTSPP/B27 with 20 ng/ml EGF and after one day, any non-adherent aggregates were discarded. Medium was changed every other day for 7-9 days.
***Step 5: Matrigel adhesion 2:*** The neurospheres/clusters which were free from fibroblast and epithelial cells were selected and picked on a new Matrigel plate. Neurospheres (not more than 10 NS) were detached and partially dissociated with 0.025% trypsin (InVitrogen) in PBS (Ca+ and Mg2+ free), for 2-3 min at 37 °C (passage 1). The trypsin was neutralized by one volume of trypsin inhibitor (Invitrogen) and four volumes of 2 mg/ml BSA in ITTSPP/B27. The cells were spun at 1200 rpm for five minutes in an Eppendorf centrifuge. The dissociated small clusters were re-seeded onto Matrigel-coated plates (at 1:1 to 1:3 ratio) in ITTSPP/B27 with 20 ng/ml EGF (step 4). After 2-3 weeks, the cells were dissociated by trypsin treatment as above (passage 2) and the resulting hES -NS cells were seeded on plates coated with poly-D-lysine (PDL, 100 µg/ml) or Matrigel coated plates and mouse laminin (20 µg/ml) or PDL (20 µg/ml) and fibronectin 5 µg/ml in ITTSPP/B27 with 20 ng/ml EGF, for one day. At each new seeding, 1 µg/ml mouse laminin (Sigma) was added to the medium.
***Steps 6 and 7: Expand on poly-D-lysine or Matrigel:*** After one day, the medium was changed to N2/B27 medium consisting of DMEM/F12 with 0.5% (v/v) N2 supplement (Invitrogen), 1% (v/v) B27 supplement. Growth factors EGF and bFGF were added at10 ng/ml each. After 7-14 days, the cells were split (1:2 or 1:3, passage 2 (P2) by trypsinization to obtain monolayers. After one week, cells were collected with trypsin and frozen in 0.5x10⁶ cells ampoule (P3). Further passages were conducted weekly, when indicated.
   In order to enrich and promote astrocyte commitment the cells may be grown on Matrigel, Cultrex or Geltrex.
***Step 8: GF removal:*** At different passages, the terminal differentiation was initiated by removing the growth factors. Whenever growth factors were removed 50 µg/ml vitamin C was added to the N2/B27 medium. Cells were thawed in N2/B27 medium supplemented with growth factors bFGF and EGF. One day before splitting and seeding in 96 well plates, growth factors were removed.

### Neuro-protective assay using astrocyte condition medium or astrocytes addition

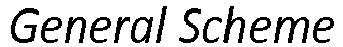

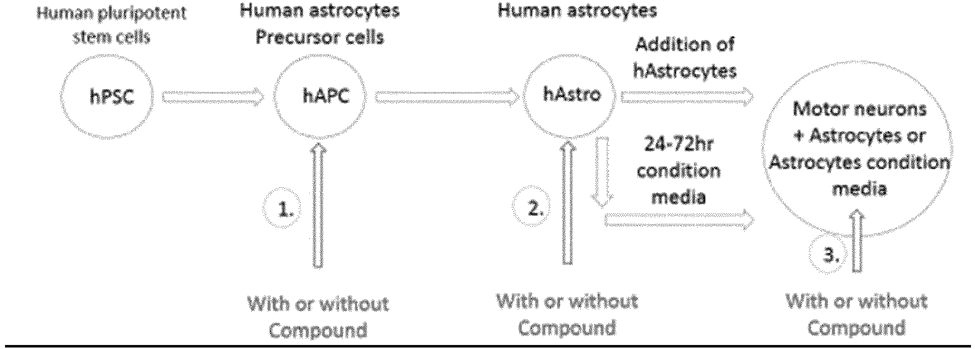

The Effect of compounds can be tested at the following steps (yellow circle):
1. Compounds can be added during hAPC differentiation toward mature astrocytes, after which the compound exposed astrocytes or their condition media can be added to the neurons.
2. At last media change for the 24-72 hrs. before inducing oxidative stress.
3. In co-culture during oxidative stress.
4. Combination can be conducted, i.e. 1+2, 1+3, 2+3 etc.

### Step 1: Generation of motor neurons from spinal cords of rodents

The spinal cords from rat embryos (E15) were used for each experiment. The meninges were removed from all spinal cords. The spinal cord tissues were chopped to small fragments and divided to 15 ml tubes (five embryos per tube). Trypsin (2 ml, 0.25%) (Gibco #250050014) was added to each tube and incubated for 15 min at 37°C. The cells were re-suspended in NB medium (7 ml) and FCS (1 ml), centrifuged at 300g for five minutes and the supernatant was aspirated. The pellet was re-suspended in NB medium (2 ml). Single cells were counted and seeded (120K cell/well) on matrigel (BD #FAL354230) coated 96 well plates (Costar #cc-3596). The cells were cultured in NB medium supplemented with 50ng/ml NGF (Alomone #n-100), with 2-day medium changes. Characterization of the motor neurons was conducted after three days of culture. The neurons were stained with Tubulin-b3, Neurofilament and HB9 (motor neuron specific transcription factor). At least 70% of the neurons were positive for these antibodies.

### Step 2: Astrocytes seeding

The astrocytes were seeded on day 8-10 (after spinal cord operation). Cells were cultured with NB medium supplemented with 50ng/ml NGF (Alomone #n-100).

### Step 3: Astrocyte condition medium generation

Medium from hPSC derived astrocytes (30-150 days old astrocytes) was collected; diluted 1:1 with fresh N2B27 supplemented with Vitamin C. The medium was named ACM (astrocyte condition medium).

### Step 4: Oxidative stress induction (conducted on day 9-10)

50µM or 150 µM H₂O₂ (Sigma # 216763) was diluted in N2B27 supplemented with Vitamin C or ACM (150µl) and added to each well of the plates seeded with the astrocytes. The plates were incubated for 6hrs at 37°C.

The plates were fixed with 4% PFA, 100µl/well for 10 minutes, and then the plates were washed twice and stored at 4°C until staining.

### Step 5: Motor neurons cell death analysis:

### Staining:

The cells were incubated with blocking buffer supplemented with Tx-100 0.3% (sigma) for 1hr at room temperature (RT), then washed with PBS (-/-).

The cells were incubated with the following Antibodies for 1hr at RT:
1^{st} Antibodies- Rb-anti Caspase-3a (Promega # G7481) (1:250)
M-anti-β-Tub3 (Convence # MMS-435P) (1:500)
Rb-anti-HB9 (Abcam # AB-ab92606) (1:100)

The cells were washed twice using PBS (-/-), and incubated with the following Antibodies for 1hr at RT:
2^{nd} Antibodies- Goat-anti-Rb-568 (Invitrogen # A11036) (1:1000)
Goat-anti-mouse-488 (Invitrogen #A11029) (1:1000)

The cells were washed twice using PBS (-/-), labeled with DAPI (1:1000), incubated for 5 min and washed twice using PBS (-/-). The HCS analysis was conducted using the Scan Array -Cellomics (Thermo-scientific inc).

The effect of ACM or addition of hPSC derived astrocytes can be tested on other stress inducers such as:
- Motor neurons excitotoxicity. Specifically, Glutamate toxicity (Glutamate uptake capacity is tested in addition to motor neurons cells viability)
- Additional reactive oxygen species toxicity
- AMPA/Kainate toxicity

### Media composition:

### N2B27 medium:

- DMEM/F12 (Sigma #01-170) 96.5%.
- N2 (Invitrogen #17502-048) 0.5%.
- B27 (Invitrogen #17504044) 1%.
- Pen/Srep/Ampho B (Biological ind. #03-033-1B) 1%.
- GlutaMAX (Invitrogen #35050038) 1%.

### NB medium:

- Neurobasal medium (Gibco # 21103049) 97%.
- B27 (Invitrogen #17504044) 1%.
- Pen/Srep/Ampho B (Biological ind. #03-033-1B) 1%.
- GlutaMAX (Invitrogen #35050038) 1%.

### Characterization of human PSC derived astrocytes using FACS analysis: Samples used for FACS:

The samples from hPSC derived astrocytes at different developmental stages were stained;
Day 0 = hPSC derived astrocytes grown in the presence of EGF and bFGF
Days 7-14= hPSC derived astrocytes grown in the absence of EGF and bFGF for 7-14 days
Days 14-28= hPSC derived astrocytes grown in the absence of EGF and bFGF for 14-28 days
Days 28-42=hPSC derived astrocytes grown in the absence of EGF and bFGF for 28-42 days
Days 42-56=hPSC derived astrocytes grown in the absence of EGF and bFGF for 42-56 days

### FACS staining:

### Reagents:

FACS buffer: PBS (Gibco #14190-094) 99.5%
BSA (sigma #A9418) 0.5%

### Antibodies:

- GLAST (Miltenyi)
- A2B5 (Miltenyi)
- CD44 (Miltenyi)
- CXCR4 (Miltenyi)
- CD140 (BD)
- CD9 (Miltenyi)
- CD133 (Miltenyi)
- TRA1-60 (Miltenyi)
- EpCAM (Miltenyi)
- ALDH1L1 (Miltenyi)
- GFAP (After fixation) (Sigma)
- Aqua4 (After fixation+Tx-100)

### Cell preparation for adherent cells:

Cells were trypsinized for 3 minutes using trypsin (Gibco), inactivated with DTI (Gibco) supplemented with 2mg/ml BSA (Sigma, Cat#: A9418) in DMEM/F12 (Sigma). Cells were collected with the media into conical tube, centrifuged at 300g for 5 minutes at RT and the supernatant was discarded. Cells were re-suspended with cold PBS Buffer (Gibco, Cat#: 14190-094), centrifuged at 300g for 5 minutes at RT and the supernatant was discarded.

### Fixation:

Cells were re-suspended with PFA 0.5% (diluted, EMC) and incubated for 30 minutes at 4°C. Cells were centrifuged at 300g for 5 minutes, the supernatant was discarded.

Cells were washed in 1 ml PBS, centrifuged at 300g for 5 minutes and the supernatant was discarded.

### Permeabilization for intracellular antigens staining:

Cells were incubated with Tx-100 0.5% for 30 minutes at 4°C to permeabilize the cell membrane. Cells were centrifuged at 300g for 5 minutes, the supernatant was discarded. Cells were re-suspended in PBS, centrifuged at 300g for 5 minutes and the supernatant was discarded.

### Immunostaining:

Cells were re-suspended with FACS Buffer (FB) (1x10⁵ cells/tube, in a volume of 100ul).

### For an unconjugated primary antibody:

The unconjugated primary antibody and the unconjugated isotype control antibody were diluted separately in FB. Primary antibody was diluted at the optimal dilution according to the manufacturer's instructions. Cells were re-suspended with the diluted primary antibody or diluted isotype control antibody and incubated for 30 min at 4°C.

Cells were centrifuged at 300g for 5 minutes and the supernatant was discarded. Cells were washed with FB and centrifuged at 300g for 5 minutes, the supernatant was discarded. The corresponding fluorochrome conjugated secondary antibodies were diluted in FB. Cells were re-suspended with the diluted secondary antibody and incubated for 30 minutes at 4 °C. The secondary antibody was diluted at the optimal dilution according to the manufacturer's instructions and this incubation must be done in the dark. Cells were re-suspended with FB and centrifuged at 300g for 5 minutes, the supernatant was discarded. Cells were re-suspended in 0.5 ml FB and analyzed on flow cytometer.

### For a fluorochrome conjugated primary antibody:

The fluorochrome conjugated primary antibody was diluted in FB according to the manufacturer's instructions. Cells were re-suspended with the conjugated primary antibody and incubated for 15 min at 4°C in dark. Cells were centrifuged at 300g for 5 minutes and the supernatant was discarded. Cells were re-suspended with FB and centrifuged at 300g for 5 minutes and the supernatant was discarded. Cells were re-suspended in 0.5 ml PBS and analyzed on flow cytometer.

### Gene expression of human PSC derived astrocytes:

### Samples used for RNA extraction:

RNA samples were extracted from hPSC derived astrocytes at different developmental stages;
Day 0 = hPSC derived astrocytes grown in the presence of EGF and bFGF
Days 7-14= hPSC derived astrocytes grown in the absence of EGF and bFGF for 7-14 days
Days 14-28= hPSC derived astrocytes grown in the absence of EGF and bFGF for 14-28 days
Days 28-42=hPSC derived astrocytes grown in the absence of EGF and bFGF for 28-42 days
Days 42-56=hPSC derived astrocytes grown in the absence of EGF and bFGF for 42-56 days

### Extraction of RNA from hPSC derived astrocytes:

0.05% Trypsin (Invitrogen#25200-056) was added to the cell sample (1^{∗}10^6-10^{∗}10^6 cells). Cells were incubated for 5 minutes at 37°C. Trypsin was inactivated by addition of 2 volumes of 2mg/ml BSA (Sigma). Cells were centrifuged at 300rpm and supernatant was removed. Pellet was re-suspended in 1ml of RLT (Qiagen) and 10µL beta-mercaptoethanol, and stored at -80C.

### Real-Time RT-PCR

### Reagents and Equipments

TaqMan® Gene Expression Assay Probes:
- A2B5 (ABI, Life technologies)
- GFAP Hs00909236 (life technologies)
- BDNF (ABI, Life technologies)
- PDGFR Hs00998018 (life technologies)
- GLAST (ABI, Life technologies)
- GDNF (ABI, Life technologies)
- ALDH1L1 Hs00201836 (life technologies)
- TRA-1-60 (ABI, Life technologies)
- Nanog Hs04260366 (life technologies)
- Oct4 (ABI, Life technologies)
- Glul Hs00365928 (life technologies)
- IGF-1(ABI, Life technologies)
- NGF (ABI, Life technologies)
- Aqua4 (ABI, Life technologies)
- Connexin30 (ABI, Life technologies)
- CD44 (ABI, Life technologies)
   - Human total RNA (Ambion).
   - Human Fetal RNA (Clontech).
   - TaqMan PCR master mix, 50ml (Applied Biosystems).
   - Fast Optical 96-well reaction plate (Applied Biosystems).
   - T-Professional basic gradient for RT-PCR (Biometra).
   - High Capacity cDNA Reverse Transcription Kit (200 reactions) (Applied Biosystems).
   - Step One Plus real time PCR system (Applied Biosystems).

### Reverse Transcription

Reverse Transcription was carried out using the High Capacity cDNA Reverse Transcription Kit (Applied Biosystems) and T-Professional basic gradient. The following procedure was based on Invitrogen's protocol.

The following RNA/primer mixture was prepared in each tube:

| | |
|---|---|
| RT Buffer | 2µl |
| Total RNA | 1 µg |
| RT random primers | 1 µl |
| RNase inhibitor | 1 µl |
| Reverse transcriptase | 1 µl |
| 100mM dNTP mix | 0.8 µl |
| DEPC H₂O | to 10 µl |

The automated thermocycler was programed for:
1. 25°C 10 min
2. 37°C 120 min
3. 85 °C 5 min
4. 4°C Pause

cDNA was stored at -20°C until use for real-time PCR.

### Real-time PCR

The following mixture was prepared in each well of optical plate (for 10 µl reaction mixture).
5 µl TaqMan Mix
0.5 µl probe+primers
0.5 µl cDNA
4 µl H₂O

Data obtained by qPCR for each of the genes were analyzed using adult fetal brain RNA as a reference.

### hPSC derived astrocytes neurotrophic factor secretion assays

### Elisa protocol:

Commercial kits used:
- BDNF (promega #G7610)
- GDNF (promega #G7620)
- IGF-1 (R&D Systems # DG100)
- VEGF (R&D Systems #DVE00)
- NGF (promega #G7630)

hPSC derived astrocytes were grown in the absence of growth factors (EGF+bFGF) for at least 20 days. hPSC derived astrocyte supernatant media (condition medium) and/or cell content (24h-72h) was collected after last media replenishing.

For each of the factors Elisa was conducted according manufacture protocol instructions.

### Glutamate uptake protocol:

Commercial kit: - EnzyChrom Glutamate Assay Kit (Bioassay Systems # EGLT-100) was used.

Cell culture samples used: at least 20 days growth factor deprived (EGF+bFGF) hPSC derived astrocytes, positive control- human astrocytes (Gibco), negative control-human fibroblasts. Glutamate (0.5-3mM) was added to each experimental well.

At least 0.5 ml of medium sample from the tested cells was taken at the following time pointes: T=0', 10', 30', 60', 90', 120' and stored them at 4°C until further processing. Glutamate uptake was conducted according to the manufacture's protocol (Bioassay Systems # EGLT-100).

### Human Astrocytes used for transplantation:

Human astrocytes were generated as described above ("Generation of the astrocytes used for the assay") going through steps 1 until 8. For transplantation, human astrocytes precursor cells were allowed to differentiate by removal of growth factors. Two cell populations were used for the experiment: one that was grown in the absence of growth factors for 7 days ("Day7") and the other for 42 days ("day42"). Human astrocytes of "day7" and "day42" were suspended in DMEM/F12 at a concentration of 2.85×10⁵ cells/µL.

### Cell Transplantation

### Experimental Design

Five transplantation experimental groups were tested. In **group #1**, 67±2 day old SOD1^{G93A} mice were transplanted with "day42" differentiated human astrocytes (n = 10 SOD1^{G93A} mice). Group#2, 67±2 day old SOD1^{G93A} mice were transplanted with "day7" differentiated human astrocytes (n=12 SOD1^{G93A} mice). Group#3, two injections of "day 7" cells were conducted on 67±2 day old SOD1^{G93A} mice and on day 97±2 SOD1^{G93A} mice (n=13). Group#4 was injected with vehicle only (DMEM/F12 Sham injected group) on day 67±2 day old SOD1^{G93A} mice (n=10) and group#5 did not received any treatment (Intact group, n=4). The mice were immunosuppressed via daily I.P. injections of 10 mg/Kg Cyclosporine (Sandimmun, Novartis) starting 3 days prior transplantation until the end of the experiment, in addition 15mg/kg CellCept (Roche) (per O.S) was given twice a day starting 3 days prior transplantation until day7 after transplantation.

### Human astrocytes Transplantation

Immune suppressed animals received transplants at 67±2 days (All groups) and 97±2 (group#3). 7µl of media or 2.0×10⁶ cells in total volume of 7µl were injected Intrathecal through the Cisterna Magna. Cells were delivered using a Hamilton Gastight syringe (10 µL) with an attached 30-gauge 45° beveled needle (Hamilton; Reno, NV). After the completion of the transplantation session, cell viability was assessed using Nucleocounter and was found to be greater than 85%.

### SOD1^{G93A} mice

Transgenic mice carrying the human SOD1 gene with the G93A mutation were used (B6SJL-Tg(SOD1^{∗}G93A)1Gur/J). Male and female mice were distributed evenly between experimental groups. Mice were obtained from The Jackson Laboratory (Bar Harbor, ME), and maintained as an in-house colony.

### Care and Treatment of Animals

All procedures were conducted in strict accordance with the Israeli guidelines; measures were taken to minimize any potential pain or animal discomfort. Mice were housed at standard temperature (21°C) and in a light controlled environment with ad libitum access to the food and water, and were maintained in racks of ventilated cages located in the same room. In order to avoid dehydration, access to cage water dishes was provided when animals started to show disease symptoms.

### Behavioral and motor performance analyses

### Forelimb Grip Strength

Animal weighing and all behavioral data collection began one week prior to transplantation, and was conducted twice weekly until end-stage. Forelimb muscle grip strengths were separately determined using a "Grip Strength Meter". Grip strength testing was performed by allowing the animals to grasp a thin bar attached to the force gauge. This was followed by pulling the animal away from the gauge until the hind- or forelimbs released the bar. This provides a value for the force of maximal grip strength. The force measurements were recorded in three separate trials, and the averages were used in analyses.

### Rotarod performance test

Motor function was conducted twice a week. Motor function was tested using an acceleration Rota-Rod device (for 180 seconds, Rota-Rod 7650; Ugo Basile, Comerio, Italy). The time by which a mouse failed from the rod was recorded. Animals were trained for one week prior to recording. Recording started one week before implantation.

### BBB- clinical scoring

Scoring is on the scale of 0 to 5, according to the table below. Mice may be given "in-between" scores (i.e. 0.5, 1.5, 2.5, 3.5) when the clinical picture lies between two defined scores.

The score was done on scale 0 to 5:

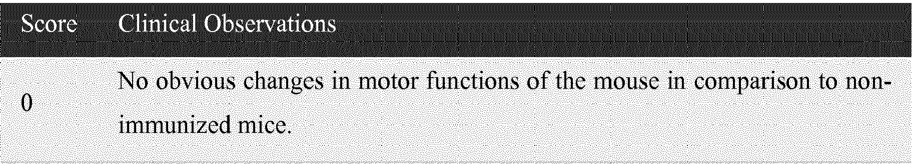

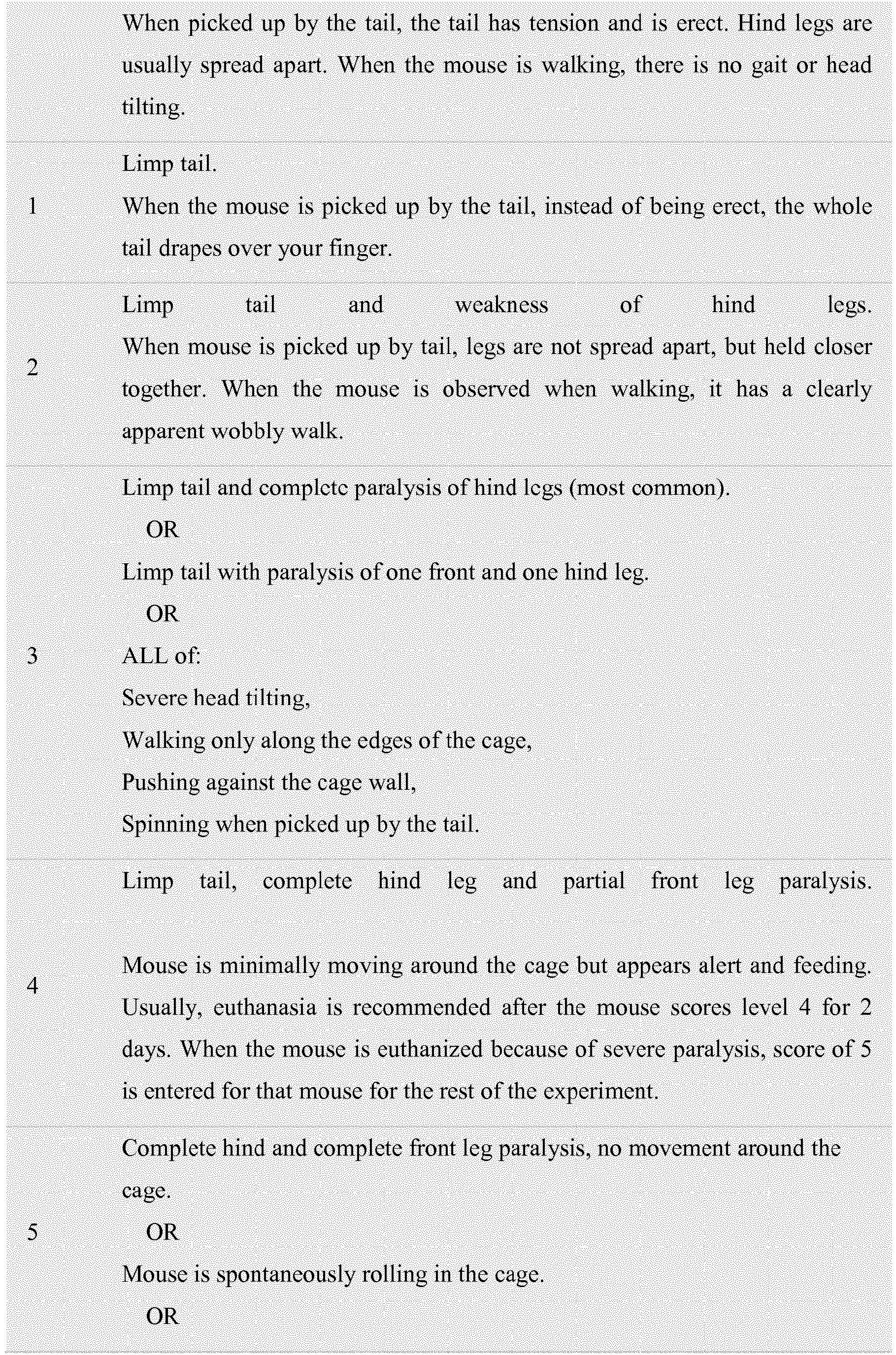

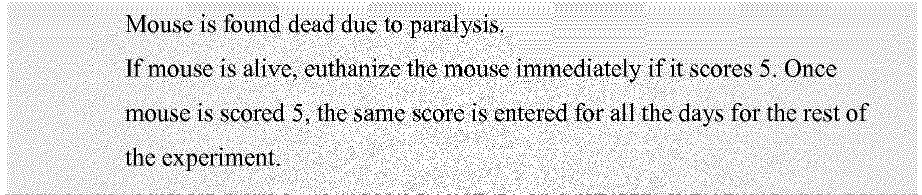

Mice are given "in-between" scores (i.e. 0.5, 1.5, 2.5, 3.5) when the clinical picture lies between two defined scores. In most cases mice reach clinical score of 3.5-4 and their clinical signs are worsening from that point forward.

### Body weight

Body weight was measure twice a week.

### Survival/Endstage and onset analysis

To determine disease end stage in a reliable and ethical fashion, endstage was defined by the inability of mice to right themselves within 30 seconds when placed on their sides.

Disease onset was defined by a decrease in rotarod performance beneath 160 seconds.

### Statistical Analyses

Kaplan-Meier analysis of the SOD1^{G93A} mice was conducted using the statistical software Sigmastat (SAS Software) to analyze survival, disease onset and duration data. Weight Rotarod, BBB and grip strength results were analyzed via repeated measures ANOVA. In some cases, Student *t-test* was performed to compare data between groups of animals. All data are presented as mean ± S.E.M., and significance level was set at p≤0.05.

### Histological and Biochemical Analyses

### Tissue Processing

Animals were sacrificed at end stage by transcardial perfusion with 0.3% saline, followed by ice-cold 4% paraformaldehyde (Fisher Scientific; Pittsburgh, PA). Spinal cords were removed from the animal, followed by cryoprotection in 30% sucrose (Fisher)/.1 M phosphate buffer at 4°C for 3 days. The tissue was embedded in OCT (Fisher), fast frozen with dry ice, and stored at -80°C until processed. Spinal cord tissue blocks were cut in the sagittal or transverse planes at 30 µm thicknesses. Sections were collected on glass slides and stored at -20°C until analyzed. Subsets of spinal cord slices were collected in PBS for free-floating histochemistry.

### Quantification of Transplant Survival and Migration

HuNA (human nuclear antigen; Millipore; Temecula, CA; monoclonal; 1:400) was used to selectively identify transplant-derived human cells (both hGRPs and hFs).

### EXAMPLE 1

### Generation and Characterization of human pluripotent stem cells (PSC) derived astrocytes in-vitro.

The differentiation of human astrocytes precursor cells (APC) toward mature astrocytes was tested for each astrocytes line in-vitro by:
a. Real time PCR (qPCR). The following probes (mRNA) were tested: BDNF, GDNF, PDGFR, GFAP, GLAST and ALDH1L1. Figure.1 demonstrated the kinetics of astrocyte lineage gene expression of hESC derived astrocytes. In order to promote astrocyte precursor cell differentiation toward mature phenotype, the growth factors were removed from the media. Samples were collected and tested on day 0 (with growth factors) and every other week upon growth factors removal. Human fetal brain was used as reference sample for all tested genes (Relative Quantification (RQ) =1) and together with human adult brain served as positive controls. It is important to note that the human adult brain consist of more than 50% astrocytes. In this study a significant increase was found in all tested astrocytes genes upon cell differentiation, and in many cases the expression of astrocytic genes (i.e. GFAP, BDNF, PDGFRα and GLAST) was significantly higher than human fetal brain (Figure. 1). These results prove that the protocol results in enriched population of human astrocytes.
b. Fluorescence-activated cell sorting (FACS, Flow cytometry) analysis using early and late glial restricted markers such as: CD44, CXCR4 and GLAST. At the presented FACS analysis (Figure. 2) the kinetics of early astrocyte markers such as CD44 and CXCR4, and GLAST as a late astrocyte marker were tested. CXCR4 also known as Fusin or CD184 is chemokine receptor with potent chemotactic activity in the CNS. It was found that above 95% of the cells expressed CXCR4 and CD44 on day 0 till 21, these levels were decreased upon astrocytes maturation. In parallel, the levels of GLAST were increased upon astrocytes maturation (Figure. 2). CXCR4 expression indicates a high migratory capacity potential of the hESC derived astrocyte precursor cells, a capacity needed for the cells to reach their destination.
c. Immunohistochemistry (IHC) using astrocyte specific antibodies: GFAP, GLAST, S100b and Aquaporin4. Staining is quantified by our ScanArray high content screening device (HCS). On Figure 3 hPSC derived astrocyte were stained on day 50 of differentiation in-vitro. As can be seen in the images, the majority of the cells are positive for astrocyte- specific markers; GFAP, GLAST and AQP4.

### EXAMPLE 2

### Test the biological functionality of the hPSC derived astrocytes

Several experiments were conducted to prove that hPSC derived human astrocytes exhibit functional properties of mature healthy astrocytes. The results in-vitro shed light on the potential in-vivo mechanism of action. For that aim the following functional mechanisms were tested:
a. **Neurotrohic factors (NTF) secretion:** Elisa for BDNF, GDNF and VEGF was conducted on astrocytes supernatant media and detergent extracts. Different astrocyte activators such as IFN-γ and LPS were used. Figure 4 demonstrated that upon activation of hPSC derived astrocytes, GDNF, BDNF and VEGF are secreted to the media (Figure. 4, middle columns respectively).
b. **Glutamate uptake:** Astrocytes glutamate uptake capacity was tested by measuring the levels of glutamate in astrocyte supernatant media. Colorimetric assay (Enzychrom) was used in order to measure the levels of glutamate in the media. Fibroblasts were used as a negative control while astrocytes from adult human tissue were served as positive control. The data has shown a glutamate uptake by human PSC derived astrocytes (Figure. 5). In this study the kinetics of glutamate uptake of two glutamate concentrations were tested (0.5mM and 2mM) at the following time points: 0', 10', 30', 60' 90' and 120' after addition of glutamate. It was found that the hPSC derived astrocytes uptake glutamate from the media in both concentrations in time dependent manner. This indicates the capacity of the specific sample of astrocytes to uptake glutamate
c. **MNs neuroprotective assay:** in this assay, mouse or human derived MNs were challenged with hydroxide peroxide (H₂O₂). The effect of hPSC derived astrocytes condition media and/or astrocytes addition on the survival of MNs was tested after induction of oxidative stress. The number of live/dead MNs was evaluated by using high content screening device (Cellomics-Scan Array). This assay can serve as valuable tool for finding new drugs that affect the survival of MNs in ALS disease. The data have shown neuroprotective effect of conditioned media from hPSC derived astrocytes tissue culture on rodent spinal cord MNs that were under oxidative stress (with H₂O₂). As shown in Figure 6, the protective effect of condition media from hPSC derived astrocytes and the effect of adding hPSC derived astrocytes directly to mouse MNs culture were tested. In two concentrations of H₂O₂ (50µM and 150µM). A significant decrease in MNs death was found after adding hPSC derived astrocytes conditioned media in both H₂O₂ concentrations. Strikingly, the neuro-protective effect of adding hPSC derived astrocytes to the MNs culture that were under oxidative stress was even greater than adding their conditioned medium (Figure. 6) suggesting that their presence is more significant in lowering the oxidative stress that merely what they secrete. These results further demonstrate in vitro the neuro-protective capacity of our hPSC derived astrocytes. The hPSC derived astrocytes protocol results in high purity of human astrocytes. The human astrocytes display functional astrocytic properties in-vitro such as; the secretion of neurotrophic factors and glutamate uptake, properties known to increase the survival of motor neurons. An in vitro model was used to directly validate the potential of hPSC derived astrocytes in protecting motor neurons under oxidative stress. This assay is a valuable tool in finding drugs that increase human astrocytes neuro-protective potential in-vivo.

## Claims

1. A method of screening an agent for preventing or treating Amyotrophic Lateral Sclerosis (ALS) the method comprising:
(a) contacting a population of astrocytes, the astrocytes having been ex-vivo differentiated from pluripotent stem cells (PSC), with the agent;
(b) co-culturing the population of astrocytes of step (a) with a population of neurons under stress selected from hypoxicity, oxidative stress, glutamate toxicity or AMPA/kainate toxicity; and
(c) quantifying an effect of said agent to enhance survival or neural function of the population of neurons.

2. The method of claim 1, wherein in step (b) the ratio of the population of astrocytes to neurons is greater than 1 : 1, 10: 1, 100: 1, 1000: 1, or 10,000: 1.

3. The method of claim 1, wherein said astrocytes
express each of GFAP, GLAST, AQP4, or a combination thereof; or
display secretion of neurotrophic factors selected from the group consisting of BDNF, GDNF and VEGF.

4. The method of claim 1, wherein the oxidative stress is selected from the group consisting of reactive oxygen species (ROS), H₂O₂, and any derivative thereof.

5. The method of claim 1, wherein said quantifying is conducted by counting the number of neurons which are under apoptosis.

6. The method of claim 5, wherein said apoptosis is detected by Caspase - 3a labeling, Annexin V, Tubulin-B3, HB9 or DAPI.

7. The method of claim 1, wherein said agent is a small molecule.

## Patentansprüche

1. Verfahren zum Screening eines Agens zum Vorbeugen oder Behandeln von Amyotropher Lateralsklerose (ALS), wobei das Verfahren umfasst:
(a) in Kontakt bringen einer Population von Astrozyten, wobei die Astrozyten aus pluripotenten Stammzellen (PSC) ex-vivo differenziert wurden, und/oder einer Population von Astrozyten-Vorläuferzellen mit dem Agens;
(b) Co-Kultivieren der Population von Astrozyten aus Schritt (a) mit einer Population von unter Stress stehenden Neuronen ausgewählt unter Hypoxie, oxidativem Stress, Glutamat-Toxizität oder AMPA/Kainat-Toxizität; und
(c) Quantifizieren eines Effektes des Agens das Überleben oder neurale Funktionen der Population von Neuronen zu verstärken.

2. Verfahren nach Anspruch 1, wobei in Schritt (b) das Verhältnis der Population von Astrozyten zu Neuronen größer ist als 1:1, 10:1, 100:1, 1000:1, oder 10.000:1.

3. Verfahren nach Anspruch 1, wobei die Astrozyten jedes von GFAP, GLAST, AQP4, oder einer Kombination daraus exprimieren; oder
eine Sekretion neurotropher Faktoren ausgewählt aus der Gruppe bestehend aus BDNF, GDNF und VEGF zeigen.

4. Verfahren nach Anspruch 1, wobei der oxidative Stress ausgewählt ist aus der Gruppe bestehend aus reaktiven Sauerstoffspezies (ROS), H₂O₂, und jedem Derivat davon.

5. Verfahren nach Anspruch 1, wobei das Quantifizieren durch Zählen der Anzahl von Neuronen durchgeführt wird, die unter Apoptose stehen.

6. Verfahren nach Anspruch 5, wobei die Apoptose durch Caspase-3a-Markierung, Annexin V, Tubulin-B3, HB9 oder DAPI erfasst wird.

7. Verfahren nach Anspruch 1, wobei das Agens ein kleines Molekül ist.

## Revendications

1. Procédé de criblage d'un agent pour la prévention ou le traitement de la sclérose latérale amyotrophique (SLA), le procédé comprenant :
(a) la mise en contact d'une population d'astrocytes, les astrocytes ayant été différenciés ex vivo à partir de cellules souches pluripotentes (CSP), avec l'agent ;
(b) la coculture de la population d'astrocytes de l'étape (a) avec une population de neurones sous un stress choisi parmi l'hypoxicité, le stress oxydatif, la toxicité au glutamate ou la toxicité à l'AMPA/kaïnate ; et
(c) la quantification d'un effet dudit agent pour améliorer la survie ou la fonction neuronale de la population de neurones.

2. Procédé selon la revendication 1, où, à l'étape (b), le rapport de la population d'astrocytes aux neurones est supérieur à 1:1, à 10:1, à 100:1, à 1000:1 ou à 10.000:1.

3. Procédé selon la revendication 1, lesdits astrocytes
exprimant chacun parmi GFAP, GLAST, AQP4 ou une combinaison correspondante ; ou
présentant une sécrétion de facteurs neurotrophiques choisis dans le groupe constitué par le BDNF, le GDNF et le VEGF.

4. Procédé selon la revendication 1, le stress oxydatif étant choisi dans le groupe constitué par des espèces réactives de l'oxygène (ROS), H₂O₂ et n'importe quel dérivé correspondant.

5. Procédé selon la revendication 1, ladite quantification étant réalisée par comptage du nombre de neurones qui sont sous apoptose.

6. Procédé selon la revendication 5, ladite apoptose étant détectée par marquage à la Caspase-3a, par l'annexine V, la tubuline-B3, HB9 ou DAPI.

7. Procédé selon la revendication 1, ledit agent étant une petite molécule.
